# EUROPEAN PATENT APPLICATION

(11) **EP 4 830 703 A2**
(43) Date of publication of application: **28.10.2026**
(21) Application number: 26190313.2
(22) Date of filing: 12.08.2020
(51) Int. Cl.: A61K 9/14

(54) **PHARMACEUTICAL COMPOSITION COMPRISING ENSIFENTRINE FOR ADMINISTRATION BY INHALATION**

(30) Priority: 12.08.2019 GB 201911517
(62) Document number(s) of the earlier application(s) in accordance with Art. 76 EPC: 20758285.9 / 4 013 384
(71) Applicant: Verona Pharma Limited, London EC2M 6UR (GB)
(72) Inventor: SPARGO, Peter Lionel, Canterbury, CT2 8NR (GB); FRENCH, Edward James, Canterbury, CT1 3LX (GB)
(74) Representative: J A Kemp LLP

(57) **Abstract**

The present invention relates to a dry powder pharmaceutical composition suitable for administration by inhalation comprising: (i) ensifentrine particles; (ii) coarse lactose particles having a Dv50 of from 40 µm to 80 µm; and (iii) fine lactose particles having a Dv50 of from 5 µm to 10 µm, wherein: the fine lactose particles are present in an amount of from 0.1 wt% to 6.0 wt% relative to the total weight to the dry powder pharmaceutical composition. Also provided is a dry powder inhaler comprising the dry powder pharmaceutical composition and medical use of the dry powder pharmaceutical composition.

## Description

### FIELD OF THE INVENTION

The present invention relates to a dry powder pharmaceutical composition comprising a respiratory drug. The invention also relates to a dry powder inhaler comprising the dry powder pharmaceutical composition.

### BACKGROUND OF THE INVENTION

Ensifentrine (9,10-dimethoxy-2-(2,4,6-trimethylphenylimino)-3-(N-carbamoyl-2-aminoethyl)-3,4,6,7-tetrahydro-2H-pyrimido[6,1-a]isoquinolin-4-one; also known as RPL554) is a dual PDE3/PDE4 inhibitor and is described in WO 00/58308 A1. As a combined PDE3/PDE4 inhibitor, ensifentrine has both anti-inflammatory and bronchodilatory activity and is useful in the treatment of respiratory disorders such as asthma and chronic obstructive pulmonary disease (COPD). The structure of ensifentrine is shown below.

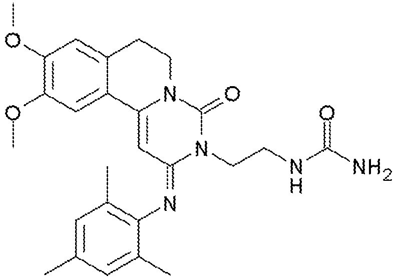

Ensifentrine is typically administered by inhalation in view of its efficacy in the treatment of respiratory disorders. Administration of ensifentrine by nebulizer is known (WO 2016/042313 A1). However, it is often desirable to administer respiratory drugs using a handheld inhaler, for example a dry powder inhaler (DPI).

It is important that a dry powder pharmaceutical formulation can be used in a DPI to deliver an appropriate dose of the active agent in an efficacious manner. Effective formulations for DPIs are typically those delivering a high fine particle fraction (FPF, corresponding to the proportion of the emitted dose having a particle size of less than 5 µm). WO 00/58308 A1 describes a dry powder formulation comprising ensifentrine and lactose. WO 2014/140647 A1 describes a dry powder composition comprising an ensifentrine/beta-adrenergic receptor antagonist combination and a blended lactose comprising at least 15 % of lactose particles having a mass median diameter (MMD) of less than 15 µm.

There is a need to develop a dry powder formulation which delivers ensifentrine effectively. It is also desirable to prepare a dry powder formulation which is capable of delivering a wide range of different ensifentrine doses.

### SUMMARY OF THE INVENTION

It is a surprising finding of the present invention that a dry powder composition comprising ensifentrine and a specific blend of lactose comprising a low proportion of fine lactose with a specific particle size, delivers an improved fine particle fraction. This composition provides an improved way in which to administer ensifentrine by inhalation.

The invention provides a dry powder pharmaceutical composition suitable for administration by inhalation comprising: (i) ensifentrine particles; (ii) coarse lactose particles having a Dv50 of from 40 µm to 80 µm; and (iii) fine lactose particles having a Dv50 of from 5 µm to 10 µm, wherein the fine lactose particles are present in an amount of from 0.1 wt% to 6.0 wt% relative to the total weight to the dry powder pharmaceutical composition.

The invention also provides a dry powder inhaler comprising a dry powder pharmaceutical composition according to the invention.

Further provided by the invention is a dry powder pharmaceutical composition according to the invention for use in the treatment of the human or animal body.

### DETAILED DESCRIPTION OF THE INVENTION

The dry powder pharmaceutical composition comprises the fine lactose particles in an amount of from 0.1 wt% to 6.0 wt% relative to the total weight to the dry powder pharmaceutical composition. Typically, the amount of the fine lactose particles is from 0.5 wt% to 5.0 wt% or from 1.0 wt% to 5.0 wt% relative to the total weight of the dry powder pharmaceutical composition. The amount of fine lactose may be from 2.0 wt% to 4.5 wt%. Preferably the fine lactose particles are present in an amount of from 3.5 wt% to 4.0 wt%, for instance at an amount of about 3.75 wt%. Alternatively, the amount of fine lactose particles may be from 0.25 wt% to 1.0 wt%, for instance about 0.5 wt%, or the amount of fine lactose particles may be from 2.0 wt% to 3.0 wt%, for instance about 2.5 wt%.

Particle sizes are described herein by reference to the Dv50 value, which is the median particle size for a volume distribution. Thus, half the volume of the particles have diameters of less than the Dv50 value and half the volume of the particles have diameters of greater than the Dv50 value. This is a well-known manner in which to describe particle size distributions. The parameters of Dv10 and Dv90 may also be used to characterise a particle size distribution of a sample. 10% of the volume of particles have a diameter of less than the Dv10 value. 90% of the volume of the particles have a diameter of less than the Dv90 value.

The technique used to measure the Dv50 (and Dv10 and Dv90) values as stated herein is typically laser diffraction. For instance, the coarse lactose particles typically have a particle size distribution with a Dv50 value of from 40 µm to 80 µm as measured by laser diffraction and the fine lactose particles have a particle size distribution with a Dv50 value of from 5 µm to 10 µm as measured by laser diffraction.

The particle size distribution of low aqueous solubility materials, for instance the ensifentrine particles, may be as measured by laser diffraction using a wet powder dispersion system. For instance, the particle size distribution can be measured by laser diffraction using a Malvern Spraytec in conjunction with a wet dispersion cell. Typically, the instrument parameters for the Malvern Spraytec are as follows:
- particle - standard opaque particle;
- refractive index Particle - 1.50;
- refractive index (imaginary) - 0.50;
- density of particle - 1.00;
- refractive index of dispersant - 1.33;
- controller unit - 1000RPM;
- measurement type - timed;
- initial sampling time - 30s;
- obscuration - 20% - 30%;
- dispersant - 1% Polysorbate 20 in deionised water.

The particle size distribution of materials soluble in water, for instance lactose particles, may be as measured by laser diffraction using a dry powder dispersion system. For instance, the particle size distribution can be measured by laser diffraction using a Malvern or Sympatec dry dispersion cell. An example of a Sympatec dry dispersion cell is a the HELOS/BR laser diffraction sensor together with a RODOS dry dispersion unit. The measurement of the particle size of lactose may be as described in United States Pharmacopoeia 34, 2011, General Chapter <429>, "Light diffraction measurement of particle size", p 161, for instance using ISO 13320:2009 Particle Size Analyses; Laser Diffraction Methods, Part 1: General Principles (2009).

The particles in the dry powder pharmaceutical composition according to the invention may be produced by any pharmaceutically acceptable size reduction process or particle size controlled production process. For instance, the particles may be produced by spray-drying a solution, by controlled crystallisation, or by size reduction of a solid form, for example by air jet milling, mechanical micronisation or media milling. The coarse and fine lactose particles may for instance be produced by jet milling of lactose.

The fine lactose particles have a Dv50 of from 5 µm to 10 µm. For instance, the fine lactose particles may have a Dv50 of from 5.0 µm to 10.0 µm. The fine lactose particles typically have a Dv10 value of from 0.5 µm to 4.0 µm, for instance from 1.0 µm to 3.0 µm. The fine lactose particles typically have a Dv90 value of less than or equal to 30 µm, for instance from 10 µm to 30 µm.

The coarse lactose particles are typically present in an amount of from 80.0 wt% to 99.0 wt % relative to the total weight of the pharmaceutical composition. For instance, the coarse lactose particles may be present in an amount of from 90.0 wt% to 96.0 wt%.

The coarse lactose particles typically have a Dv50 of from 50 µm to 70 µm, preferably from 55 µm to 65 µm. For instance, the coarse lactose particles may have a Dv50 of about 60 µm.

The coarse lactose particles may comprise at least 95 wt% lactose or may consist essentially of lactose. The coarse lactose particles typically consist of lactose. The fine lactose particles may comprise at least 95 wt% lactose or may consist essentially of lactose. The fine lactose particles typically consist of lactose.

A composition which consists essentially of a component typically comprises only that component and other components which do not materially affect the essential characteristics of the component of which the composition essentially consists. A composition consisting essentially of a component may comprise at least 99.5 wt% of that component relative to the total weight of the composition.

The dry powder pharmaceutical composition comprises a blend comprising the coarse lactose particles and the fine lactose particles. The particle size distribution of the lactose blend will accordingly be bimodal and comprise two peaks, one peak corresponding to the modal particle size of the coarse lactose particles and one peak corresponding to the modal particle size of the fine lactose particles. As the skilled person will appreciate, there may be some overlap between particle size distributions of the coarse and fine lactose particles in the lactose blend. For the avoidance of doubt, the lactose blend present in the dry powder pharmaceutical composition is obtainable by mixing said coarse lactose particles having a Dv50 of from 40 µm to 80 µm and said fine lactose particles having a Dv50 of from 5 µm to 10 µm.

The ensifentrine particles are typically present in an amount of from 0.1 wt% to 20 wt% relative to the total weight of the dry powder pharmaceutical composition. The amount of ensifentrine particles is preferably from 0.1 wt% to 10 wt%. For instance, the ensifentrine particles may be present in an amount of from 0.2 wt% to 6.0 wt%. The amount of ensifentrine particles may be from 2.0 wt% to 4.5 wt% or from 3.5 wt% to 4.0 wt%. For instance, the amount of ensifentrine particles may be about 2.5 wt% or about 3.75 wt% relative to the total weight of the dry powder pharmaceutical composition. Alternatively, the amount of the ensifentrine particles may be from 0.1 wt% to 0.5 wt%, for instance about 0.25 wt%.

Typically, the amount (wt%) of the ensifentrine particles present in the dry powder pharmaceutical composition is from 40% to 120% of the amount (wt%) of the fine lactose particles present in the dry powder pharmaceutical composition. For instance, the amount (wt%) of the ensifentrine particles may be from 90% to 110% of the amount (wt%) of the fine lactose particles present. In such a case, if 3.75 wt% of fine lactose particles were present, the amount of ensifentrine particles may be from 3.375 wt% (90%) to 4.125 wt% (110%).

The ensifentrine particles comprise ensifentrine (i.e. ensifentrine free base) or a pharmaceutically acceptable salt thereof. Typically, the ensifentrine particles comprise ensifentrine. The ensifentrine particles typically comprise at least 90.0 wt% of ensifentrine or a pharmaceutically acceptable salt thereof, more preferably at least 95.0 wt%. The ensifentrine particles may consist essentially of ensifentrine or a pharmaceutically acceptable salt thereof, or may consist of ensifentrine or a pharmaceutically acceptable salt thereof. For instance, the ensifentrine particles may consist of ensifentrine free base.

In some cases, the dry powder pharmaceutical composition comprises less than 0.1 wt% of a second active agent, which second active agent is a muscarinic receptor antagonist or a beta-adrenergic receptor antagonist. For instance, the dry powder pharmaceutical composition may be free of a second active agent, which second active agent is a muscarinic receptor antagonist or a beta-adrenergic receptor antagonist. In a preferred embodiment, ensifentrine is the sole active agent in the dry powder pharmaceutical composition of the invention.

The ensifentrine particles typically have a Dv50 of from 0.5 µm to 5.0 µm. The ensifentrine particles preferably have a Dv50 of from 1.0 µm to 2.0 µm. Typically, the Dv10 of the ensifentrine particles is from 0.2 µm to 1.0 µm and the Dv90 of the ensifentrine particles is from 3.0 µm to 6.0 µm. For instance, the Dv10 of the ensifentrine particles may be from 0.4 µm to 0.6 µm and the Dv90 of the ensifentrine particles may be from 3.2 µm to 3.8 µm.

The dry powder pharmaceutical composition may contain additional excipients. Typically, however, the major components of the dry powder pharmaceutical composition are the ensifentrine particles, the coarse lactose particles and the fine lactose particles. For instance, the total amount of the ensifentrine particles, the coarse lactose particles and the fine lactose particles is typically at least 90.0 wt% relative to the total weight of the dry powder pharmaceutical composition. Preferably the dry powder pharmaceutical composition comprises at least 95.0 wt% of the ensifentrine particles, the coarse lactose particles and the fine lactose particles relative to the total weight of the dry powder pharmaceutical composition. The dry powder pharmaceutical composition may consist essentially of, or consist of, the ensifentrine particles, the coarse lactose particles and the fine lactose particles.

The dry powder pharmaceutical composition may comprise: (i) the ensifentrine particles in an amount of from 0.1 wt% to 5.0 wt%; (ii) the coarse lactose particles in an amount of from 92.0 wt% to 99.5 wt%; and (iii) the fine lactose particles in an amount of from 0.1 wt% to 5.0 wt%, wherein the amounts are relative to the total weight of the dry powder pharmaceutical composition.

The dry powder pharmaceutical composition may comprise: (i) the ensifentrine particles in an amount of from 0.1 wt% to 0.4 wt%; (ii) the coarse lactose particles in an amount of from 99.0 wt% to 99.5 wt%; and (iii) the fine lactose particles in an amount of from 0.25 wt% to 0.75 wt%.

The dry powder pharmaceutical composition may comprise: (i) the ensifentrine particles in an amount of from 2.0 wt% to 3.0 wt%; (ii) the coarse lactose particles in an amount of from 94.0 wt% to 96.0 wt%; and (iii) the fine lactose particles in an amount of from 2.0 wt% to 3.0 wt%.

The dry powder pharmaceutical composition may comprise: (i) the ensifentrine particles in an amount of from 3.5 wt% to 4.0 wt%; (ii) the coarse lactose particles in an amount of from 92.0 wt% to 93.0 wt%; and (iii) the fine lactose particles in an amount of from 3.5 wt% to 4.0 wt%.

The dry powder pharmaceutical composition is typically suitable for administration by a dry powder inhaler. For instance, the dry powder pharmaceutical composition may be suitable for administration by a capsule dry powder inhaler, a blister dry powder inhaler or a reservoir dry powder inhaler.

The dry powder pharmaceutical composition may be produced by standard formulation methods. The dry powder pharmaceutical composition may for instance be produced by a method comprising mixing the ensifentrine particles, the coarse lactose particles and the fine lactose particles. The components may be mixed using a high shear mixer.

The invention provides a dry powder inhaler (DPI) comprising a dry powder pharmaceutical composition as defined herein. The DPI may be a blister DPI, a capsule DPI or a reservoir DPI. DPIs are well known to those of ordinary skill in the art, and many such devices are commercially available, with representative dry powder inhaler devices including Aerolizer^{™} (Novartis), Airmax^{™} (IV AX), ClickHaler^{™} (Innovata Biomed), Diskhaler^{™} (GlaxoSmithKline), Diskus^{™} or Accuhaler (GlaxoSmithKline), Easyhaler^{™} (Orion Pharma), Eclipse^{™} (Aventis), FlowCaps^{™} (Hovione), Handihaler^{™} (Boehringer Ingelheim), Pulvinal^{™} (Chiesi), Rotahaler^{™} (GlaxoSmithKline), SkyeHaler^{™} or Certihaler^{™} (SkyePharma), Twisthaler (Schering-Plough), Turbuhaler^{™} (AstraZeneca), Ultrahaler^{™} (Aventis), Plastiape RS01 Dry Powder Inhaler (RPC), Powdair (Hovione), MRX003 (Merxin) and MRX001 (Merxin).

The dry powder pharmaceutical composition is useful in the treatment of the human or animal body. Ensifentrine is useful in the treatment of respiratory diseases and inflammatory diseases.

The invention provides a dry powder pharmaceutical composition as defined herein for use in the treatment or prevention of a disease or condition selected from asthma, allergic asthma, hay fever, allergic rhinitis, bronchitis, emphysema, bronchiectasis, chronic obstructive pulmonary disease (COPD), adult respiratory distress syndrome (ARDS), steroid resistant asthma, severe asthma, paediatric asthma, cystic fibrosis, lung fibrosis, pulmonary fibrosis, interstitial lung disease, skin disorders, atopic dermatitis, psoriasis, ocular inflammation, cerebral ischaemia, inflammatory diseases and auto-immune disease. Typically, the dry powder composition is for use in treating COPD or asthma. Prefereably, the disease or condition is COPD.

Typically, the dry powder pharmaceutical composition is administered by inhalation from a DPI. The dry powder pharmaceutical composition may be administered by a DPI device to provide a metered nominal dose of from 5 µg to 1000 µg of ensifentrine per inhalation. For instance, the metered nominal dose per inhalation may be from 10 µg to 500 µg.

Typically, the emitted dose from a DPI comprising the dry powder pharmaceutical composition is from 70% to 95% of the metered nominal dose.

Typically, the dry powder pharmaceutical composition is administered to the patient via 1 to 8 inhalations of the DPI per day. For instance, the pharmaceutical composition may administered to the patient by 1 or 2 inhalations up to four times per day.

The invention also provides a dry powder pharmaceutical composition as defined herein for use in a method of treating a disease or condition as defined herein, which method comprises inhalation (or actuation) of a dry powder inhaler comprising the dry powder pharmaceutical composition, wherein the fine particle fraction of the ensifentrine particles following inhalation (or actuation) is at least 20%. The fine particle fraction of the ensifentrine particles following inhalation is typically at least 30% and preferably at least 35%. The fine particle fraction may be at least 40%.

The fine particle fraction (FPF) is the fraction of the emitted dose of ensifentrine particles having an aerodynamic particle size of less than 5 µm. The emitted dose is the total amount of ensifentrine particles emitted from a dry powder inhaler device comprising the dry powder pharmaceutical composition.

Methods for measuring the fine particle fraction are well known to the skilled person. For example, the FPF may be measured by cascade impaction techniques, such as Andersen Cascade Impactor or by Next Generation Impactor. Typically, the fine particle fraction of the ensifentrine dry powder formulation is measured using a Next Generation Impactor.

The invention also provides a method of treating or preventing a disease or condition as defined herein in a subject, which method comprises administering to said subject an effective amount of a dry powder pharmaceutical composition as defined herein.

The invention is described in more detail by the following Examples.

### EXAMPLES

### Materials and methods

The following materials were used.
- Micronized ensifentrine (Dv50: 1.3 µm)
- Coarse lactose 1 (Dv50: 60 µm)
- Coarse lactose 2 (Dv50: 75 µm)
- Coarse lactose 3 (Dv50: 105 µm)
- Fine lactose 1 (Dv50: < 5 µm)
- Fine lactose 2 (Dv50: < 10 µm)

The particle size distributions of these materials is set out in Table 1.

**Table 1**

| Material | Dv10 (µm) | | Dv50 (µm) | | Dv90 (µm) | |
|---|---|---|---|---|---|---|
| Micronized ensifentrine | 0.5 | | 1.3 | | 3.6 | |
| Coarse lactose 1 | 30 | | 60 | | 90 | |
| Coarse lactose 2 | 10 | | 75 | | 140 | |
| Coarse lactose 3 | 50 | | 105 | | 175 | |
| Fine lactose 1 | not measured | | < 5 | | ≤ 10 | |
| Fine lactose 2 | 1-3 | | < 10 | | < 30 | |

Dry powder compositions were produced by the following method.
1. The lactose grades were passed through a 450 µm sieve.
2. The total amounts of fine and coarse lactose particles were weighed.
3. The lactose grades were added to the vessel of a high-shear mixer and mixed for 5 minutes.
4. The total amount of ensifentrine was sieved and weighed.
5. Two thirds of the lactose blend obtained in step 3 was removed.
6. The total amount of ensifentrine was added to the high-shear mixer vessel and then one of the two thirds of the lactose blend that was removed in step 5 was added to the composition.
7. The composition was mixed for five minutes.
8. The final one of the two thirds of the blend of lactose grades that was removed in step 5 was added to the composition.
9. The composition was mixed for five minutes.
10. The resulting mixed dry powder composition was transferred to a polyethylene bag.

### Example 1 - comparison of formulations

Formulations comprising various lactose carriers and differing amounts of ensifentrine were produced and filled into capsules for testing with a capsule dry powder inhaler. The blend uniformity (percentage of label claim (%LC) and relative standard deviation (RSD)), content uniformity (%LC and RSD) and aerodynamic profile of the formulations were assessed. The aerodynamic profile included measurement of non-emitted fraction, emitted dose (ED), fine particle dose (FPD), fine particle fraction (FPF) and mass balance.

First, three formulations were produced comprising each of coarse lactose 1 (Dv50: 60 µm), coarse lactose 2 (Dv50: 75 µm) and coarse lactose 3 (Dv50: 105 µm). The compositions (as weight percent) and characteristics of these formulations are shown in Table 2.

| *Table 2* | **Formulation** | **1** | | **2** | | **3** | |
|---|---|---|---|---|---|---|---|
| Composition | Ensifentrine (%) | 2.5 | | 2.5 | | 2.5 | |
| | Coarse lactose (%) | 97.5 | | 97.5 | | 97.5 | |
| | Coarse lactose Dv50 (µm) | 60 | | 105 | | 75 | |
| | Fine lactose (%) | 0 | | 0 | | 0 | |
| | Fine lactose Dv50 (µm) | - | | - | | - | |
| Blend uniformity | Assay (%LC) | 92.7 | | 95.2 | | 93.7 | |
| | %RSD | 0.2 | | 7.0 | | 1.2 | |
| | Assay (%LC) | 91.3 | | 90.2 | | 96.7 | |
| Content uniformity | %RSD | 0.7 | | 0.6 | | 1.1 | |
| | | Average | %RSD | Average | %RSD | Average | %RSD |
| | Non-emitted fraction (µg) | 53.9 | 1.6 | 56.3 | 2.7 | 88.9 | 13.4 |
| Aerodynamic profile | ED (µg) | 407.3 | 0.1 | 404.5 | 3.2 | 404.1 | 2.4 |
| | FPD (µg) | 197.2 | 3.8 | 155.5 | 2.2 | 163.8 | 7.6 |
| | FPF_{ED} (cut off 5 µm) (%) | 48.4 | 3.9 | 38.5 | 5.3 | 40.5 | 6.0 |
| | Mass balance (%) | 92.2 | 0.1 | 92.2 | 2.6 | 98.6 | 2.3 |

It was found that formulation 1 comprising coarse lactose 1 (Dv50: 60 µm) had the greatest FPF and ED. Coarse lactose 1 was accordingly selected as the preferred coarse lactose.

Next, formulations comprising a blend of coarse lactose 1 and either fine lactose 1 (Dv50 < 5 µm) or fine lactose 2 (Dv50 < 10 µm) were produced. The composition and characteristics of these formulations are shown in Table 3.

| *Table 3* | **Formulation** | **4** | | **5** | |
|---|---|---|---|---|---|
| Composition | Ensifentrine (%) | 2.5 | | 2.5 | |
| | Coarse lactose (%) | 93.75 | | 93.75 | |
| | Coarse lactose Dv50 (µm) | 60 | | 60 | |
| | Fine lactose (%) | 3.75 | | 3.75 | |
| | Fine lactose Dv50 (µm) | < 10 | | < 5 | |
| Blend uniformity | Assay (%LC) | 97.6 | | 96.2 | |
| | %RSD | 0.9 | | 2.2 | |
| Content uniformity | Assay (%LC) | 97.7 | | 96.3 | |
| | %RSD | 0.8 | | 0.3 | |
| Aerodynamic profile | | Average | %RSD | Average | %RSD |
| | Non-emitted fraction (µg) | 64.8 | 0.0 | 74.3 | 0.0 |
| | ED (µg) | 406.0 | 1.1 | 396.0 | 2.4 |
| | FPD (µg) | 191.0 | 2.5 | 176.0 | 2.4 |
| | FPF_{ED} (cut off 5 µm) (%) | 47.0 | 2.1 | 45.0 | 0.5 |
| | Mass balance (%) | 94.4 | 0.3 | 96.2 | 2.3 |

It was found that the addition of fine lactose 2 (with a Dv50 of < 10 µm) unexpectedly increased the FPF relative to the addition of fine lactose 1 (with a smaller Dv50). It was therefore found that a combination of coarse lactose 1 and fine lactose 2 produced improved aerodynamic properties.

Compositions comprising different proportions of coarse lactose 1 (Dv50: 60 µm) and fine lactose 2 (Dv50 < 10 µm) were then assessed, and the results are shown in Table 4.

| *Table 4* | **Formulation** | **6** | | **7** | | **8** | |
|---|---|---|---|---|---|---|---|
| Composition | Ensifentrine (%) | 0.25 | | 0.25 | | 0.25 | |
| | Coarse lactose (%) | 99.75 | | 96.0 | | 92.25 | |
| | Coarse lactose Dv50 (µm) | 60 | | 60 | | 60 | |
| | Fine lactose (%) | 0.0 | | 3.75 | | 7.50 | |
| | Fine lactose Dv50 (µm) | - | | < 10 | | < 10 | |
| Blend uniformity | Assay (%LC) | 104.2 | | 97.2 | | 103.5 | |
| | %RSD | 0.9 | | 2.3 | | 4.5 | |
| Content uniformity | Assay (%LC) | 106.6 | | 107.8 | | 108.1 | |
| | %RSD | 3.7 | | 1.0 | | 1.2 | |
| Aerodynamic profile | | Average | %RSD | Average | %RSD | Average | %RSD |
| | Non-emitted fraction (µg) | 9.5 | 7.2 | 9.7 | 7.1 | 11.4 | 7.3 |
| | ED (µg) | 51.6 | 13.6 | 51.3 | 1.7 | 47.0 | 1.3 |
| | FPD (µg) | 18.4 | 30.8 | 18.2 | 5.5 | 14.8 | 2.2 |
| | FPF_{ED} (cut off 5 µm) (%) | 34.7 | 3.0 | 35.8 | 3.1 | 31.8 | 3.4 |
| | Mass balance (%) | 123.8 | 9.7 | 121.3 | 1.7 | 116.3 | 2.1 |

It was found that addition of 3.75 wt% of the fine lactose 2 increased the FPF, but the FPF then unexpectedly decreased as the proportion of fine lactose 2 was increased up to 7.50 wt %. The analysis was repeated for compositions comprising a greater proportion of ensifentrine (5 wt%) and the results are shown in Table 5.

| *Table 5* | **Formulation** | **9** | | **10** | | **11** | |
|---|---|---|---|---|---|---|---|
| Composition | Ensifentrine (%) | 5 | | 5 | | 5 | |
| | Coarse lactose (%) | 95 | | 91.25 | | 87.5 | |
| | Coarse lactose Dv50 (µm) | 60 | | 60 | | 60 | |
| | Fine lactose (%) | 0 | | 3.75 | | 7.5 | |
| | Fine lactose Dv50 (µm) | - | | < 10 | | < 10 | |
| Blend uniformity | Assay (%LC) | 98.5 | | 96.0 | | 99.8 | |
| | %RSD | 0.5 | | 2.5 | | 0.3 | |
| Content uniformity | Assay (%LC) | 95.4 | | 93.3 | | 97.5 | |
| | %RSD | 0.7 | | 1.3 | | 0.9 | |
| Aerodynamic profile | | Average | %RSD | Average | %RSD | Average | %RSD |
| | Non-emitted fraction (µg) | 142.3 | 4.0 | 126.8 | 6.6 | 119.2 | 1.6 |
| | ED (µg) | 846.9 | 0.2 | 830.7 | 0.6 | 878.4 | 1.4 |
| | FPD (µg) | 402.6 | 4.8 | 452.1 | 5.4 | 446.3 | 2.1 |
| | FPF_{ED} (cut off 5 µm) (%) | 46.9 | 3.8 | 54.0 | 6.5 | 51.6 | 2.2 |
| | Mass balance (%) | 100.0 | 1.6 | 96.5 | 2.0 | 98.4 | 2.1 |

The same pattern was observed for the formulations comprising 5 wt% ensifentrine as for those comprising 0.25 wt% ensifentrine, with the highest FPF obtained when 3.75 wt% of fine lactose 2 was used. The FPF again decreased as the proportion of fine lactose was increased to 7.50 wt%.

It has therefore been found that a blend of lactose comprising a *reduced* amount (around 3.75 wt%) of fine lactose can be used to deliver an increased FPF for dry powder compositions comprising ensifentrine. The improved FPF is maintained at both the 0.25 wt% ensifentrine and 5 wt% ensifentrine compositions.

### Example 2 - stability assessment

Formulations 12 to 14 according to the invention were produced with the compositions set out in Table 6. Each blended formulation was filled into size #3 hypromellose capsules with a net fill weight of 20 mg/capsule.

| *Table 6* | **Formulation** | **12** | **13** | **14** |
|---|---|---|---|---|
| | Ensifentrine (%) | 0.25 | 2.50 | 3.75 |
| Composition | Coarse lactose (%) | 99.25 | 95.0 | 92.5 |
| | Fine lactose (%) | 0.50 | 2.50 | 3.75 |

The stability of formulations 12, 13 and 14 was assessed over 24 months under the conditions of 25 °C and 60% RH. It was observed that each formulation remained stable over the 24 month period. No major changes were observed in the assay or in the aerodynamic performance of the formulations.

The stability of formulations 12, 13 and 14 was also assessed under accelerated conditions of 40 °C and 75% RH. Good stability was observed after at least 6 months of storage under these conditions.

### Example 3 - clinical assessment

A 2-part, 37 patient, Phase II single and repeat dose clinical study with ensifentrine in patients with COPD was completed using DPI formulations 12, 13 and 14 (Table 6).

Single doses of ensifentrine up to 3 mg had a rapid, dose-dependent, and statistically significant bronchodilatory effect up to the 3 mg dose level over 12 hours. Ensifentrine doses of 0.15, 0.5, 1.5, and 3 mg provided dose-dependent improvements from baseline in peak Forced Expiratory Volume in one second (FEV₁) (over 4 hours) of up to 333 mL compared with placebo (p<0.01 for 1.5 mg and 3 mg doses).

The second part of the study was a complete block crossover study using the same patients that were dosed in the first part of the study. Ensifentrine doses of 0.15 mg to 3 mg dosed twice daily over 7 days also provided dose-dependent, highly statistically significant and clinically meaningful improvements from baseline in peak FEV₁ (over 4 hours) on Day 7 of 102, 175, 180, and 260 mL, respectively, compared with placebo (p≤0.0001 for all dose groups). The improvement in FEV₁ was sustained over the 12 hour dosing interval as shown by clinically meaningful and statistically significant improvements in average FEV₁ over 12 hours of up to 147 mL (p<0.05 for all dose groups) and in morning trough FEV₁ on Day 7 of 98, 87, and 97 mL, respectively, compared with placebo (p≤0.001 for each dose).

The following are additional aspects of the invention.
1. A dry powder pharmaceutical composition suitable for administration by inhalation comprising:
   (i) ensifentrine particles;
   (ii) coarse lactose particles having a Dv50 of from 40 µm to 80 µm; and
   (iii) fine lactose particles having a Dv50 of from 5 µm to 10 µm,
   wherein:
   the fine lactose particles are present in an amount of from 0.1 wt% to 6.0 wt% relative to the total weight to the dry powder pharmaceutical composition.
2. A dry powder pharmaceutical composition according to aspect 1, wherein the amount of fine lactose particles is from 0.5 wt% to 5.0 wt% relative to the total weight of the dry powder pharmaceutical composition, preferably from 3.5 wt% to 4.0 wt%.
3. A dry powder pharmaceutical composition according to aspect 1 or 2, wherein the coarse lactose particles are present in an amount of from 80.0 wt% to 99.0 wt % relative to the total weight of the dry powder pharmaceutical composition, preferably from 90.0 wt% to 96.0 wt%.
4. A dry powder pharmaceutical composition according to any one of the preceding aspects, wherein the coarse lactose particles have a Dv50 of from 50 µm to 70 µm, preferably from 55 µm to 65 µm.
5. A dry powder pharmaceutical composition according to any one of the preceding aspects, wherein the ensifentrine particles are present in an amount of from 0.1 wt% to 10 wt% relative to the total weight of the dry powder pharmaceutical composition, preferably from 0.2 wt% to 6.0 wt%.
6. A dry powder pharmaceutical composition according to any one of the preceding aspects, wherein the ensifentrine particles comprise at least 90 wt% ensifentrine or a pharmaceutically acceptable salt thereof relative to the total weight of the ensifentrine particles, preferably at least 95.0 wt%.
7. A dry powder pharmaceutical composition according to any one of the preceding aspects, wherein the ensifentrine particles have a Dv50 of from 0.5 µm to 5.0 µm, preferably from 1.0 µm to 2.0 µm.
8. A dry powder pharmaceutical composition according to any one of the preceding aspects, wherein the total amount of the ensifentrine particles, the coarse lactose particles and the fine lactose particles is at least 90.0 wt% relative to the total weight of the dry powder pharmaceutical composition, preferably at least 95.0 wt%.
9. A dry powder pharmaceutical composition according to any one of the preceding aspects, which dry powder pharmaceutical composition is suitable for administration by a dry powder inhaler.
10. A dry powder inhaler comprising a dry powder pharmaceutical composition according to any one of the preceding aspects.
11. A dry powder pharmaceutical composition according to any one of aspect 1 to 9 for use in the treatment of the human or animal body.
12. A dry powder pharmaceutical composition according to any one of aspects 1 to 9 for use in the treatment or prevention of a disease or condition selected from asthma, allergic asthma, hay fever, allergic rhinitis, bronchitis, emphysema, bronchiectasis, chronic obstructive pulmonary disease (COPD), adult respiratory distress syndrome (ARDS), steroid resistant asthma, severe asthma, paediatric asthma, cystic fibrosis, lung fibrosis, pulmonary fibrosis, interstitial lung disease, skin disorders, atopic dermatitis, psoriasis, ocular inflammation, cerebral ischaemia, inflammatory diseases and auto-immune disease.
13. A dry powder pharmaceutical composition for use according aspect 12 wherein the disease or condition is chronic obstructive pulmonary disease (COPD).
14. A dry powder pharmaceutical composition according to any one of aspects 1 to 9 for use in a method of treating a disease or condition as defined in aspect 12 or aspect 13, which method comprises inhalation of a dry powder inhaler comprising the dry powder pharmaceutical composition,
   wherein the fine particle fraction of the ensifentrine particles following inhalation is at least 20%.
15. A dry powder pharmaceutical composition for use according to aspect 14, wherein the fine particle fraction of the ensifentrine particles following inhalation is at least 30%, preferably at least 35%.
16. A method of treating or preventing a disease or condition as defined in aspect 12 or aspect 13 in a subject, which method comprises administering to said subject an effective amount of a dry powder pharmaceutical composition according to any one of aspects 1 to 9.

## Claims

1. A dry powder pharmaceutical composition suitable for administration by inhalation comprising:
(i) ensifentrine particles;
(ii) coarse lactose particles having a Dv50 of from 40 µm to 80 µm; and
(iii) fine lactose particles having a Dv50 of from 5 µm to 10 µm,
wherein:
the fine lactose particles are present in an amount of from 0.1 wt% to 6.0 wt% relative to the total weight to the dry powder pharmaceutical composition.

2. A dry powder pharmaceutical composition according to claim 1, wherein the amount of fine lactose particles is from 0.5 wt% to 5.0 wt% relative to the total weight of the dry powder pharmaceutical composition, preferably from 3.5 wt% to 4.0 wt%.

3. A dry powder pharmaceutical composition according to claim 1 or 2, wherein the coarse lactose particles are present in an amount of from 80.0 wt% to 99.0 wt % relative to the total weight of the dry powder pharmaceutical composition, preferably from 90.0 wt% to 96.0 wt%.

4. A dry powder pharmaceutical composition according to any one of the preceding claims, wherein the coarse lactose particles have a Dv50 of from 50 µm to 70 µm, preferably from 55 µm to 65 µm.

5. A dry powder pharmaceutical composition according to any one of the preceding claims, wherein the ensifentrine particles are present in an amount of from 0.1 wt% to 10 wt% relative to the total weight of the dry powder pharmaceutical composition, preferably from 0.2 wt% to 6.0 wt%.

6. A dry powder pharmaceutical composition according to any one of the preceding claims, wherein the ensifentrine particles comprise at least 90 wt% ensifentrine or a pharmaceutically acceptable salt thereof relative to the total weight of the ensifentrine particles, preferably at least 95.0 wt%.

7. A dry powder pharmaceutical composition according to any one of the preceding claims, wherein the ensifentrine particles have a Dv50 of from 0.5 µm to 5.0 µm, preferably from 1.0 µm to 2.0 µm.

8. A dry powder pharmaceutical composition according to any one of the preceding claims, wherein the total amount of the ensifentrine particles, the coarse lactose particles and the fine lactose particles is at least 90.0 wt% relative to the total weight of the dry powder pharmaceutical composition, preferably at least 95.0 wt%.

9. A dry powder pharmaceutical composition according to any one of the preceding claims, which dry powder pharmaceutical composition is suitable for administration by a dry powder inhaler.

10. A dry powder inhaler comprising a dry powder pharmaceutical composition according to any one of the preceding claims.

11. A dry powder pharmaceutical composition according to any one of claims 1 to 9 for use in the treatment of the human or animal body.

12. A dry powder pharmaceutical composition according to any one of claims 1 to 9 for use in the treatment or prevention of a disease or condition selected from asthma, allergic asthma, hay fever, allergic rhinitis, bronchitis, emphysema, bronchiectasis, chronic obstructive pulmonary disease (COPD), adult respiratory distress syndrome (ARDS), steroid resistant asthma, severe asthma, paediatric asthma, cystic fibrosis, lung fibrosis, pulmonary fibrosis, interstitial lung disease, skin disorders, atopic dermatitis, psoriasis, ocular inflammation, cerebral ischaemia, inflammatory diseases and auto-immune disease.

13. A dry powder pharmaceutical composition for use according claim 12 wherein the disease or condition is chronic obstructive pulmonary disease (COPD).

14. A dry powder pharmaceutical composition according to any one of claims 1 to 9 for use in a method of treating a disease or condition as defined in claim 12 or claim 13, which method comprises inhalation of a dry powder inhaler comprising the dry powder pharmaceutical composition,
wherein the fine particle fraction of the ensifentrine particles following inhalation is at least 20%.

15. A dry powder pharmaceutical composition for use according to claim 14, wherein the fine particle fraction of the ensifentrine particles following inhalation is at least 30%, preferably at least 35%.
